# EUROPÄISCHE PATENTANMELDUNG

(11) **EP 1 977 643 A1**
(43) Veröffentlichungstag der Anmeldung: **08.10.2008**
(21) Anmeldenummer: 07006838.2
(22) Anmeldetag: 02.04.2007
(51) Int. Cl.: A01N 1/02, A61K 31/192, A61K 31/216, A61K 31/7028, A61K 35/00, A61P 37/02, A61P 1/00

(54) **Herstellung einer viablen, lagerfähigen Wurmeiersuspension**

(71) Anmelder: DR. FALK PHARMA GMBH, D-79108 Freiburg (DE)
(72) Erfinder: Wilhelm, Rudolf Dr., 76476 Bischweier (DE); Roepstorff, Allan Knud, 2610 Rodovre (DK); Kapel, Christian Moliin Outzen, 2960 Rungstetd Kyst (DK)
(74) Vertreter: Keller, Günter

(57) **Zusammenfassung**

Die vorliegende Anmeldung beschreibt ein neues, vorteilhaftes Reinigungsverfahren, das die Viabilität einer Wurmeiersuspension sicherstellt.

Im nicht-embryonierten Zustand werden die T. suis Wurmeier zunächst in einer Schwefelsäurelösung bei etwa pH 2 gelagert. Hierbei werden Bakterien und Viren erfolgreich abgetötet. Die Keimzahl von Hefen und Pilzen wird reduziert.

In einem zweiten Schritt wird der pH-Wert auf 4 angehoben und ein Konservierungsmittel zugesetzt. Hierbei werden dann Hefen und Pilze erfolgreich abgetötet. Das im zweiten Reinigungsschritt venivendetet Suspensionsmedium kann dann für die weitere Herstellung (Embryonierung) und Lagerung als Kulturmedium verwendet werden.

## Beschreibung

Die vorliegende Erfindung betrifft ein Reinigungsverfahren zur Herstellung von viablen, entwicklungsfähigen Suspensionen, die Eier von parasitären Helminthen enthalten, und zur therapeutischen Anwendung geeignet sind.

Der Einfluss von Parasiteninfektionen auf die Aktivierung des Immunsystems ihrer tierischen Wirte ist bekannt (Review, D M McKay, Parasitology 2006, 132: 1-12). Von dieser Aktivierung wird auch das Vorkommen und der Verlauf von Krankheiten des Immunsystems beeinflusst. Epidemiologische Studien zeigen, dass in Regionen mit hohen Raten von Wurminfektionen Autoimmunerkrankungen seltener sind, als in Regionen in denen auf Grund besserer hygienischer Verhältnisse diese Infektionsraten niedriger sind. Cytokinprofile von Patienten mit Morbus Crohn, einer chronisch entzündlichen Darmerkrankung, haben gezeigt, dass Th2-Immunzellen durch Helminthen Infektionen stimuliert werden können. Morbus Crohn, eine Th1-dominierte Autoimmunerkrankung kann durch eine Helminthen Infektion verhindert bzw. beeinflusst werden (Summers et al., Am J Gastroenterol 2003, 98: 2034-2041). Auch andere durch Th1-Immunzellen hervorgerufene Erkrankungen, wie auch durch Helicobacter verursachte Gastritis und autoimmune Enzephalomyelitis sind beeinflussbar.

Schon 1973 berichtete Beer (Parasitology 1973, 67: 253-262), dass *Trichuris suis* ein geeigneter Nematode sein könnte, um eine Immunisierung beim Menschen zu erreichen, ohne dass eine pathogene Nematodeninfektion erfolgt. *Trichuris suis* ist eng verwandt mit *Trichuris humanis* und überlebt im menschlichen Gastrointestinaltrakt ohne sich jedoch zu vermehren. Therapeutische Massnahmen sind während dieser selbst limitierten Infektion nicht notwendig. Eine Infektion durch *Trichuris suis* kann daher eine Immunisierung bewirken.

R.J.S. Beer (Parasitology 1972, 65: 343-350) beschreibt eine Methode zum Sammeln und Reinigen von Wurmeiern. Wurmeier im embryonisierten Stadium werden in einer 0.2%igen Kaliumdichromatlösung bei 32°C kultiviert und täglich belüftet. In DE 101 63 115 wird eine ähnliche Reinigungsmethode beschrieben. Es werden Wurmeier mit Hilfe von Hydroxyl- oder Sauerstoffradikalen produzierenden chemischen Reaktionen von eventuell vorhandenen Mikroorganismen und Viren befreit. Besonders die sogenannte Fenton-Reaktion wird beschrieben. Bei dieser entsteht unter Einfluss von FeCl₂ aus H₂O₂ Sauerstoff in statu nascendi, dessen desinfizierende Wirkung genutzt wird.

Summers und Mitarbeiter (GUT 2005, 54: 87-90) reinigen T. suis-Eier ebenfalls mit einer 0.2%igen Dichromatlösung in Phosphatpuffer bei pH 6-7, nachdem sie zunächst 5-6 Wochen in einer Pufferlösung, die Antibiotika (Penicillin/Streptomycin/Amphotericin B) enthält, embryonisiert wurden.

Die Verwendung solcher Bakterien und Viren abtötender Agentien hat verschiedene wesentliche Nachteile:

Sowohl bei der Verwendung einer Dichromatlösung wie auch bei der Verwendung einer Fentonlösung muss diese nach kurzer Zeit entfernt werden, um eine Schädigung der Wurmeier zur vermeiden. Versuche zeigten zudem, dass während der nach der Reinigung erfolgenden Embryonisierung (3 Monate bei 22°C - 25°C) trotz dieser vorherigen Reinigung, eine starke Verkeimung auftritt. Auch während der anschließenden Lagerung der embryonisierten Eier bei 2-8°C kann eine Verkeimung nicht vermieden werden. Daher ist es wahrscheinlich, dass nach Entfernung solcher Mikroorganismen und Viren abtötender Agentien ein erneutes Wachstum von Keimen in den T. suis-Eisuspension erfolgt.

Zudem ist die restlose Entfernung von Dichromat aus der Lösung unbedingt notwendig. Dies ist jedoch sehr aufwendig.

Der Zusatz von Antibiotika zur Vermeidung einer starken Verkeimung im Embryonisierungsstadium der Eier wie von Summers beschrieben, verhindert zwar ein starkes Keimwachstum in diesem Stadium, ist aber nicht in der Lage, die Keime sicher abzutöten. Nach der Entfernung von Antibiotika in den nächsten Reinigungsschritten und der Lagerung bis zur Anwendung tritt erneut starkes Keimwachstum auf.

Eine Aufgabe der vorliegenden Erfindung ist es, eine Wurmeiersuspension herzustellen, die von kontaminierenden Viren und Bakterien gereinigt wird und die ohne Wachstum von Pilzsporen und Hefen gelagert werden kann. Das hier beschriebene Verfahren erhält die Entwicklungsfähigkeit der Wurmeier zu adulten Würmern. Die Wurmeiersuspension ist für eine therapeutische Anwendbarkeit geeignet.

Im Rahmen der vorliegenden Erfindung wurde überraschend gefunden, dass sich zur Lösung der beschriebenen Aufgabe ein kombiniertes Reinigungs- und Konservierungsverfahren besonders eignet, da für dieses Verfahren im Gegensatz zu den bisher bekannten Verfahren keine toxischen Substanzen (z.B. Kaliumdichromat, K₂Cr₂O₇) und keine Desinfektionsmittel verwendet werden müssen.

Während des Reinigungsverfahren werden isolierte Eier von Helminthen, vorzugsweise von T. suis, in einer Säurelösung, vorzugsweise eine H₂SO₄-Lösung, mit einem pH-Wert ≤ 2 für die Dauer von 1-72 Std. inkubiert (siehe Beispiel 1). Die optimale Inkubationszeit ist 2 bis 8 Stunden, bevorzugt 3 Stunden. Die Inkubation erfolgt bei einer Temperatur von 4°C bis 37°C, bevorzugt 25°C bis 35°C.

Bei dem Verfahren zur Herstellung einer oral verabreichbaren pharmazeutischen Präparation enthaltend eine lagerfähige Suspension von lebensfähigen Eiern von parasitären, nicht human pathogenen Helminthen, bei der sich nach Einnahme eine ausreichende Zahl an Helminthen entwickelt, die zu einer Stimulation regulatorischer humaner T-Zellen führt, wird zunächst die Suspension der Helmintheneier in einem ersten Schritt einer Säurebehandlung bei einem pH-Wert von ≤ 2 unterzogen. In einem weiteren Schritt wird der pH-Wert auf ≥ 4 angehoben und ein pharmakologisch annehmbares Konservierungsmittel zugefügt. Grundsätzlich ist es auch möglich, zuerst das Konservierungsmittel zuzugeben und dann die Säurebehandlung durchzuführen. Bevorzugt wird aber zuerst die Säurebehandlung durchgeführt und dann das Konservierungsmittel zugegeben.

Bevorzugt wird die Säurebehandlung durch Zugabe von Schwefelsäure bewirkt. Bei der Säurebehandlung wird eine ausreichende Menge an Säure zugegeben, um den pH-Wert in einen Bereich zwischen etwa 0,5 und 2 abzusenken. Geeignete Säuren sind Salzsäure, Salpetersäure oder bevorzugt Schwefelsäure. Die Säurebehandlung darf nur über einen verhältnismäßig kurzen Zeitraum erfolgen, damit die Wurmeier nicht zu stark geschädigt werden. Hier besteht auch ein Zusammenhang zwischen Konzentration an Säure und Dauer der Säurebehandlung. Bevorzugt wird der pH-Wert auf weniger als 2, bevorzugt 1 bis 2 abgesenkt. Die Dauer der Säurebehandlung beträgt einige Minuten bis wenige Stunden, bevorzugt 120 Minuten bis 360 Minuten.

Nach der Säurebehandlung wird der pH-Wert durch Zugabe einer geeigneten Base, beispielsweise NaOH, wieder auf einen pH-Wert > 4 erhöht. Anschließend wird ein oder mehrere Konservierungsmittel zugegeben, wobei grundsätzlich sämtliche für die Konservierung von Lebensmitteln oder Pharmazeutika geeignete Stoffe eingesetzt werden können.

Bevorzugt wird das Konservierungsmittel ausgewählt aus der Gruppe bestehend aus Sorbinsäure, Benzoesäure, Salzen dieser Säuren, Parabenzoesäureester, Propylenglykol oder Kombinationen dieser Konservierungsmittel.

Besonders bevorzugt eingesetzt wird ein oder mehrere der Konservierungsmittel ausgewählt aus der Gruppe bestehend aus
Sorbinsäure in einer Konzentration von 0,1 bis 0,2 Gew.-%,
Benzoesäure in einer Konzentration von 0,1 bis 0,3 Gew.-%,
p-Hydroxy-Benzoesäureester in einer Konzentration von 0,02 bis 0,3 Gew.-%, Propylenglykol in einer Konzentration von 5 bis 20 Gew.-% oder eine Kombination der genannten Konservierungsmittel in den angegebenen Konzentrationsbereichen.

Darüber hinaus können der Suspension gegebenenfalls weitere pharmazeutisch verträgliche Zusatzstoffe wie Farbstoffe, Geschmacksstoffe oder Verdickungsmittel zugefügt werden.

Gegenstand der Erfindung sind daher oral verabreichbare pharmazeutische Präparationen enthaltend eine lagerfähige Suspension von lebensfähigen Eiern von parasitären, nicht human pathogenen Helminthen, wobei sich nach Einnahme der Suspension eine ausreichende Zahl an Helminthen entwickelt, die zu einer Stimulation regulatorischer T-Zellen führt, und wobei die Suspension weniger als 1000 Kolonie-bildende (cfu = colony forming unit) Mikroorganismen pro ml Suspension beinhaltet. Bevorzugt enthält die oral verabreichbare pharmazeutische Präparation weniger als 100 Kolonie-bildende Einheiten an Mikroorganismen pro ml Suspension. Besonders bevorzugt enthält die verabreichbare pharmazeutische Präparation weniger als 10 Kolonie-bildende Einheiten an Mikroorganismen pro ml Suspension. Die Anzahl der Kolonie-bildenden Einheiten wird mit üblichen mikrobiologischen Verfahren bestimmt.

Unter Mikroorganismen versteht man hier Bakterien, Viren, Pilze, Hefen und Protozoen, wobei solche Mikroorganismen nicht vorhanden sein dürfen, die nachteilige gesundheitliche Auswirkungen haben können.

Durch das erfindungsgemäße Verfahren ist es möglich, die Suspension von lagerfähigen Eiern von parasitären, nicht humanpathogenen Helminthen in einer Form bereitzustellen, die für die pharmazeutische Verwendung geeignet sind. Durch die erfindungsgemäße Behandlung werden einerseits kontaminierende Mikroorganismen, insbesondere Bakterien, aber auch Pilze, Viren sowie gegebenenfalls Protozoen so weit abgetötet, dass das Präparat zum pharmazeutischen Einsatz geeignet ist. Andererseits ist aber die Behandlung so schonend, dass die Wurmeier ihre Fähigkeit beibehalten, nach Einnahme im Darm des Patienten heranzuwachsen, damit die gewünschte Stimulierung des Immunsystems erfolgen kann.

Die erfindungsgemäßen Präparate sind insbesondere zur Behandlung von verschiedenen entzündlichen Darmerkrankungen, vor allem chronisch entzündlichen Darmerkrankungen geeignet. In besonders vorteilhafter Weise können die Präparate zur Behandlung von entzündlichen Darmerkrankungen eingesetzt werden, die als Morbus Crohn bezeichnet werden.

### Beispiel 1: Abreicherung von Keimen in einer T. suis - Eisuspension:

In einer *Trichuris suis* Eisuspension (TSO) in Phosphatpuffer (pH 7) in einer Konzentration von 2.400 Eiern/ml wurde nach den Angaben im DAB (deutsches Arzneibuch, Abschnitt 2.6.12, zuletzt aktualisiert mit der 24. Ergänzungslieferung in 2006) die Keimzahl bestimmt. Es wurde ein Keimstatus von 190 000 cfu/ml gefunden. Diese Suspension wurde dann mit verdünnter H₂SO₄-Lösung auf pH 2 umgepuffert. Nach 3 h Lagerung bei 30°C war die Keimzahl auf < 20 cfu/ml reduziert worden.

Die Güte des vorgestellten Verfahrens wurde dann überprüft. Hierzu wurden definierte Teststämme von Bakterien, Hefen und Pilzen nach den Vorgaben des deutschen Arzneibuches (DAB) verwendet. In den Beispielen 2a, 2b und 2c sind die Ergebnisse dieser Prüfung dargestellt.

### Beispiel 2: Bestimmung der Keimzahl

a. Eine Nährsuspension entsprechend DAB 2.6.13 wurde mit den in Tab. 1 vorgegebenen sporenbildenden und nicht-sporendbildenden Testbakterien beimpft. Anschliessend wurde mit einer verdünnten H₂SO₄-Lösung auf pH 2 umgepuffert und 6 h bei 30°C gelagert. Die darauf folgende Keimzahlbestimmung ergab die Werte wie in Tab. 1 dargestellt.

**Tab. 1**

| Keim | T₀ | H₂SO₄ pH 2 nach 6 h |
|---|---|---|
| *Clostridium sporog.* | 8 000 000 | < 10 |
| *Bacillus subtilis* | 1 400 000 | 20* |
| *Escherichia coli* | 130 000 | < 10 |
| *Staphylococus aureus* | 800 000 | < 10 |
| *Salmonella typhymurium* | 250 000 | < 10 |

| | | |
|---|---|---|
| * Durch eine längere Behandlung mit Säure für ca. 6 bis 10 Stunden konnte die Anzahl der Kolonie-bildenden Einheiten auf weniger als 10 reduziert werden. | | |

b. Eine Nährsuspension entsprechend DAB 2.6.13 wurde mit den in Tab. 2 vorgegebenen Testhefen beimpft. Anschließend wurde mit einer verdünnten H₂SO₄-Lösung auf pH 2 bzw. pH 1 umgepuffert und 3 h bzw. 48 h bei 25°C gelagert. Die darauf folgende Bestimmung ergab die Werte wie in Tab. 2 dargestellt.

**Tab. 2**

| Keim | T₀ | T₃ₕ | T₄₈ₕ | T₃ₕ | T₄₈ₕ |
|---|---|---|---|---|---|
| | H₂SO₄ pH 2 | | | H₂SO₄ pH 1 | |
| Saccharomyces cer. | 470 000 | 380 000 | 220 000 | 39 000 | 40* |
| Candida albicans | 690 000 | 380 000 | 32 000 | 26 000 | <10 |

| | | | | | |
|---|---|---|---|---|---|
| * Durch eine längere Behandlung mit Säure bis zu einer Dauer von 72 Stunden konnte die CfU auf 5 reduziert werden. | | | | | |

c. Eine Nährsuspension entsprechend DAB 2.6.13 wurde mit den in Tab. 3 vorgegebenen Testschimmel beimpft. Anschließend wurde mit einer verdünnten H₂SO₄-Lösung auf pH 2, pH 1 und pH 0 umgepuffert und 3 h bzw. 48 h bei 25°C gelagert. Die darauf folgende Bestimmung ergab die Werte wie in Tab. 3 dargestellt.

**Tab. 3**

| Keim | T₀ | T₃ₕ | T₄₈ₕ | T₃ₕ | T₄₈ₕ | T₃ₕ | T₄₈ₕ |
|---|---|---|---|---|---|---|---|
| | H₂SOₐ pH 2 | | | H₂SO₄ pH 1 | | H₂SO₄ pH 0 | |
| Penicillium brevicomp. | 490 000 | 90 000 | 32 000 | 39 000 | 14 000 | 70 | <10 |
| Aspergillus niger | 800 000 | 370 000 | 700 000 | 500 000 | 500 000 | 50 | <10 |

Wenn durch die Säurebehandlung die CfU bei bestimmten Keimen nicht hinreichend reduziert wurde, wurde die Dauer der Säurebehandlung entsprechend verlängert auf bis zu 72 Stunden.

Die Beispiele 1 und 2 a-c zeigen, dass nach kurzer Zeit Bakterien mit einer verdünnten H₂SO₄-Lösung pH 2 effektiv abgetötet werden. Hefen und Pilze benötigen entweder eine längere Inkubationszeit oder stärker saure pH-Werte um effektiv abgetötet zu werden.

Die beschriebenen Reinigungsschritte sind überraschend auch zur Herstellung einer virenfreien T. suis-Eisuspension geeignet.

### Beispiel 3: Abreicherung von Viren:

Inaktivierung von Murine Leukemia Virus (MuLV), Pseudorabies Virus (PRV), Porcine Parvovirus (PPV) und Feline Calicivirus (FCV) bei der Herstellung von *Trichuris suis* Eisuspension (TSO). Es handelt sich bei den eingesetzten Viren um Modellviren.

Der Virustiter in den beimpften Proben wurde durch Endpunkttitration bestimmt. In diesem an sich bekannten Verfahren werden verdünnte Proben auf Mikrotiterplatten, die leicht zu indentifizierende Indikatorzellen enthalten, aufgebracht. Nach dieser Infektion der Indikatorzellen mit den virushaltigen Proben verändert sich durch die Zell-Lysis die Morphologie der Zellen. Diese Zell-Lyse ist unter dem Mikroskop leicht zu messen.

Die getesteten Viren, umhüllte und nicht-umhüllte, wurden in hoher Konzentration in eine 0.01 N H₂SO₄-Lösung (pH 2, 30°C), die außerdem TSO enthält, gegeben. Nach 10 min., 3 h und 72 h wurde die Aktivität der Viren bestimmt. Nach 10 min. waren die umhüllten Viren PRV und MuLV, nach 3 h die nicht-umhüllten Viren FCV und PPV nicht mehr infektiös. Die Verwendung einer mit 1 N NaOH neutralisierten Gegenprobe zeigte auch nach 3 h Inkubation, dass alle 4 Viren noch virulent waren. Wegen der Cytotoxizität einer 0,2%igen Kaliumdichromatlösung in 0,01 N Schwefelsäure war eine Testung dieser Lösung als Vergleich nicht möglich.

Nur lange Inkubation und die Absenkung des pH-Wertes auf einen pH-Wert von 0 ermöglichten eine deutliche Reduktion auch von Pilzen und Hefen (siehe Beispiel 2, Tabelle 2 und 3). Dieses bereits neue Verfahren der Aufreinigung in saurem Medium erfordert lange Inkubationszeiten, um eine deutliche Verminderung der Keimzahlen zu erreichen und hat den Nachteil, dass eine solch lange Inkubationszeit die Überlebensfähigkeit einer Wurmeiersuspension beeinträchtigt.

Als zweites Merkmal der vorliegenden Erfindung wurde nun überraschend gefunden, dass die notwendige lange Inkubationszeit in den beschriebenen Versuchen durch die Addition von üblicherweise zur Konservierung verwendeten chemischen Substanzen deutlich reduziert werden konnte. Zudem ermöglichte die Verwendung von Konservierungsmitteln die Inaktivierung von kontaminierenden Mikroorganismen schon bei pH 4. Als bevorzugte Konservierungsmittel haben sich Benzoesäure, Sorbinsäure, deren Salze und Propylenglykol herausgestellt (siehe Beispiel 4 a - e).

### Beispiel 4: Bestimmung der Koloniezahl verschiedener Mikroorganismen

Anheben des pH-Wertes von pH 2 auf pH 4 und Zusatz von Konservierungsmitteln

Versuchsbedingungen für die Beispiele 4a bis 4e:
Die getesteten Proben enthielten je 10.000 TSO/ml (± 10 %) in Phosphat-Puffer von pH 4 und entweder 0,2 % Benzoesäure, 0,1 % Sorbinsäure oder 15 % Propylenglykol.
Den Testproben wurden folgende Keime zugesetzt:
- Aspergillus niger als Einzelkeim
- Pseudomonas aruginosa, Staphylococcus aureus, Candida albicans und Escherichia coli in Kombination.

Die Inkubationstemperatur betrug 25°C. Die Testplatten wurden nach 1 bis 7 Tagen ausgewertet. Es wurden folgende Ergebnisse erhalten:

### 4 a Pseudomonas aeruginosa

| Konservierungsmittel | T 0 (cfu/ml) | T 1d (cfu/ml) | T 7d (cfu/ml) |
|---|---|---|---|
| Benzoesäure 0,2% | 180.000 | <10 | < 10 |
| Sorbinsäure 0, 1 % | 180.000 | 30 | < 10 |
| Propylenglykol 15% | 180.000 | 10 | < 10 |

### 4 b Staphylococcus aureus

| Konservierungsmittel | T 0 (cfu/ml) | T 1d (cfu/ml) | T 7d (cfu/ml) |
|---|---|---|---|
| Benzoesäure 0,2% | 130.000 | < 10 | < 10 |
| Sorbinsäure 0,1 % | 130.000 | 240 | < 10 |
| Propylenglykol 15% | 130.000 | 14.000 | < 10 |

### 4 c Escherichia coli

| Konservierungsmittel | T 0 (cfu/ml) | T 1d (cfu/ml) | T 7d (cfu/ml) |
|---|---|---|---|
| Benzoesäure 0,2% | 140.000 | < 10 | < 10 |
| Sorbinsäure 0, 1 % | 140.000 | 490 | < 10 |
| Propylenglykol 15% | 140.000 | 7.100 | < 10 |

### 4 d Candida albicans

| Konservierungsmittel | T 0 (cfu/ml) | T 1d (cfu/ml) | T 7d (cfu/ml) |
|---|---|---|---|
| Benzoesäure 0,2% | 240.000 | < 10 | < 10 |
| Sorbinsäure 0,1 % | 240.000 | 190 | < 10 |
| Propylenglykol15% | 240.000 | 8.600 | 30 |

### 4 e Aspergillus niger

| Konservierungsmittel | T 0 (cfu/ml) | T 1d (cfu/ml) | T 7d (cfu/ml) |
|---|---|---|---|
| Benzoesäure 0,2% | 120.000 | 90 | < 10 |
| Sorbinsäure 0,1% | 120.000 | 20.000 | < 10 |
| Propylenglykol15% | 120.000 | 30.000 | 9.000 |

Wenn eine starke Kontamination mit Pilzen vorliegt, werden bevorzugt Sorbinsäure, Benzoesäure und/oder p-Hydroxy-Benzoesäureester eingesetzt oder Propylenglycol mit diesen Konservierungsstoffen kombiniert eingesetzt.

Ein weiterer Vorteil der kombinierten Inaktivierung von Mikroorganismen mittels verdünnter Säuren und der Verwendung von Konservierungsmitteln ist, dass während der weiteren Verwendung der TSO-Suspension eine mögliche Rekontaminierung mit neuen Keimen, verhindert werden kann. Das Wachstum von neuen Keimen wird durch das beschriebene kombinierte Verfahren der Inaktivierung und Konservierung einer TSO-Suspension vermieden.

Die Vorbereitung zur weiteren Herstellung erfordert eine Anhebung des pH-Wertes auf einen Bereich von pH 2 - 7, vorzugsweise pH 4 - 6. Auch für diesen dritten Schritt ist die Addition von geeigneten Konservierungsmitteln erforderlich. Zu verwendende Konservierungsmittel müssen für die orale Anwendung geeignet sein. Als geeignet haben sich hierbei Sorbinsäure in einer Konzentration von 0,1 - 0,2%, Benzoesäure in einer Konzentration von 0,1 - 0,3%, p-Hydroxy-Benzoesäureester in Konzentrationen von 0,02 - 0,3%, oder Propylenglykol in einer 5 -20 %igen wässrigen Lösung erwiesen. Ebenfalls geeignet sind Gemische der oben genannten Konservierungsmittel.

Überraschend wurde gefunden, dass durch dieses Reinigungsverfahren die Viabilität der Wurmeiersuspension nicht beeinträchtigt wird.

Die in diesem Verfahren bevorzugte Reihenfolge der Reinigungsschritte kann variiert werden, ohne dass die Effektivität des Reinigungsverfahren beeinträchtigt wird.

## Patentansprüche

1. Verfahren zur Herstellung einer oral verabreichbaren pharmazeutischen Präparation enthaltend eine lagerfähige Suspension von lebensfähigen Eiern von parasitären, nicht humanpathogenen Helminthen, wobei sich nach Einnahme der Suspension eine ausreichende Zahl an Helminthen entwickelt, die zu einer Stimulation regulatorischer humaner T-Zellen führt, **dadurch gekennzeichnet, dass** die Suspension der Helmintheneier in einem Schritt einer Säurebehandlung bei einem pH-Wert von ≤ 2 unterzogen wird und, dass in einem weiteren Schritt der pH-Wert auf ≥ 4 eingestellt wird und ein pharmakologisch annehmbares Konservierungsmittel zugefügt wird.

2. Verfahren nach Anspruch 1, **dadurch gekennzeichnet, dass** die Säurebehandlung durch Zugabe von Schwefelsäure bewirkt wird.

3. Verfahren nach einem der vorhergehenden Ansprüche, **dadurch gekennzeichnet, dass** das Konservierungsmittel ausgewählt ist aus der Gruppe bestehend aus Sorbinsäure, Benzoesäure, Salzen dieser Säuren, p-Benzoesäureester, Propylenglykol oder eine Kombination dieser Konservierungsmittel.

4. Verfahren nach Anspruch 3, **dadurch gekennzeichnet, dass** eines oder mehrere der Konservierungsmittel ausgewählt aus der Gruppe bestehend aus
Sorbinsäure in einer Konzentration von 0,1 bis 0,2 Gew.-%,
Benzoesäure in einer Konzentration von 0,1 bis 0,3 Gew.-%,
p-Hydroxy-Benzoesäureester in einer Konzentration von 0,02 bis 0,3 Gew.-%, Propylenglykol in einer Konzentration von 5 bis 20 Gew.-% oder eine Kombination der genannten Konservierungsmittel in den angegebenen Konzentrationsbereichen zugegeben wird.

5. Verfahren nach einem der vorhergehenden Ansprüche, **dadurch gekennzeichnet, dass** der Suspension gegebenenfalls weitere pharmazeutisch verträgliche Zusatzstoffe zugefügt werden.

6. Oral verabreichbare pharmazeutische Präparation enthaltend eine lagerfähige Suspension von lebensfähigen Eiern von parasitären, nicht humanpathogenen Helminthen, wobei sich nach Einnahme der Suspension eine ausreichende Zahl an Helminthen entwickelt, die zu einer Stimulation regulatorischer T-Zellen führt, **dadurch gekennzeichnet, dass** die Suspension weniger als 1000 Kolonie-bildende Mikroorganismen pro ml Suspension beinhaltet.

7. Oral verabreichbare pharmazeutische Präparation nach Anspruch 6, **dadurch gekennzeichnet, dass** die Suspension weniger als 100 Kolonie-bildende Einheiten an Mikroorganismen pro ml Suspension enthält.

8. Oral verabreichbare pharmazeutische Präparation nach Anspruch 7, **dadurch gekennzeichnet, dass** die Suspension weniger als 10 Kolonie-bildende Einheiten an Mikroorganismen pro ml Suspension enthällt.

9. Oral verabreichbare pharmazeutische Präparation nach Anspruch 6 bis 8, **dadurch gekennzeichnet, dass** die Mikroorganismen ausgewählt sind aus der Gruppe bestehend aus Bakterien, Viren, Pilze, Hefen und Protozoen.

10. Pharmazeutische Präparation nach einem der Ansprüche 6 bis 9, **dadurch gekennzeichnet, dass** sie nach einem der Verfahren gemäß Anspruch 1 bis 5 herstellbar ist.

11. Verwendung einer lagerfähigen Suspension von Eiern parasitärer Helminthen hergestellt nach einem Verfahren gemäß einem der Ansprüche 1 bis 5 zur Herstellung einer pharmazeutischen Präparation zur Behandlung von entzündlichen Darmerkrankungen, insbesondere chronisch entzündlichen Darmerkrankungen.

12. Verwendung nach Anspruch 11, **dadurch gekennzeichnet, dass** es sich bei der entzündlichen Darmerkrankung um Morbus Crohn handelt.
